# EUROPEAN PATENT APPLICATION

(11) **EP 4 549 580 A1**
(43) Date of publication of application: **07.05.2025**
(21) Application number: 23831882.8
(22) Date of filing: 28.06.2023
(51) Int. Cl.: C12Q 1/6806, C12N 15/10, C40B 50/08

(54) **RNA SEQUENCING LIBRARY CONSTRUCTION METHOD**

(30) Priority: 28.06.2022 KR 20220079165; 27.06.2023 KR 20230082825
(71) Applicant: Xenohelix. Co., Ltd., Incheon 21984 (KR)
(72) Inventor: CHO, Seok Keun, Incheon 21982 (KR); LEE, Bora, Gimpo-si, Gyeonggi-do 10077 (KR)
(74) Representative: Stolmár & Partner Patentanwälte PartG mbB
(86) International application number: PCT/KR2023/008988
(87) International publication number: WO 2024/005523

(57) **Abstract**

The present invention relates to a method for constructing an RNA library, which includes ligating of a first adapter nucleic acid molecule and a second adapter nucleic acid molecule. Additionally, the method of the present invention, by including a guide nucleic acid molecule, can increase the ligation efficiency of the adapter nucleic acid molecules, thereby increasing the efficiency of RNA library construction.

## Description

### TECHNICAL FIELD

The present invention relates to a method for constructing an RNA library, which includes a step of ligating a first adapter nucleic acid molecule and a second adapter nucleic acid molecule. Furthermore, by adding a guide nucleic acid molecule, the ligation efficiency of the adapter nucleic acid molecules can be increased, thereby increasing the efficiency of constructing an RNA library.

### BACKGROUND ART

A genome refers to all the genetic information possessed in an organism. For sequencing or sequence analysis of an individual's genome, various technologies such as DNA chips, next generation sequencing (NGS), and next-next generation sequencing (NNGS) are being developed. NGS is widely used for research and diagnostic purposes. NGS varies depending on the type of equipment, and in general, it may be divided into three stages: collection of a sample, construction of a library, and analysis of a nucleic acid sequence. After the analysis of a nucleic acid sequence, various genetic information such as presence of a genetic mutation, can be confirmed based on the sequence analysis data generated.

In particular, as the quality of life improves, there is a growing interest in early diagnosis of diseases, and since molecular diagnostic technology directly detects the genetic information (DNA/RNA) of pathogens that cause diseases, it has been receiving much attention as a technology that can solve the shortcomings of immunodiagnostic technology that detects indirect factors of diseases based on existing antigen/antibody reactions.

The construction of a library through exploration and acquiring disease-related genetic information can be used to discover candidate substances for new drug development, and thus, there is an increasing demand for technology development that allows faster and more accurate library construction.

### [Prior Art Document]

(Patent Document 1) KR Patent Application Publication No. 10-2021-0141915

### DISCLOSURE OF THE INVENTION

### TECHNICAL PROBLEM

An object of the present invention is to provide a method for constructing an RNA library, which includes a first adapter nucleic acid molecule and a second adapter nucleic acid molecule.

Another object of the present invention is to provide a composition for constructing an RNA library, which includes a first adapter nucleic acid molecule, a second adapter nucleic acid molecule, and a guide nucleic acid molecule strand.

Still another object of the present invention is to provide an RNA library constructed by the method described above.

### TECHNICAL SOLUTION

In order to achieve the above-described objects, an aspect of the present invention relates to a method for constructing an RNA library, in which the method includes: a) ligating a first adapter nucleic acid molecule to the 3' end of a target RNA; b) synthesizing cDNA through complementary strand polymerization of the molecule ligated in the step a); c) removing RNA by treating with RNase; d) ligating a second adapter nucleic acid molecule to the 3' end of the cDNA; and e) PCR amplifying the nucleic acid molecule ligated in the step d).

Specifically, the target RNA may be a small RNA. The small RNA may refer collectively to RNA consisting of about 50 nucleotides or less, in addition to miRNAs and siRNAs.

As used herein, "a first adapter nucleic acid molecule" refers to a nucleic acid molecule that is ligated to a target RNA, and a phosphate group at the 5' end of the first adapter nucleic acid molecule recognizes and binds to the 3' end of a target RNA. Then, complementary strand polymerization may be performed using a molecule, in which a first adapter nucleic acid molecule is ligated to the 3' end of the target RNA, as a template.

As used herein, the "complementary strand polymerization" refers to the polymerization of a sequence complementary to a molecule ligated to the 3' end of a target RNA using the molecule as a template. One of these complementary strand polymerization methods may include reverse transcription but is not limited thereto, and any sequence complementary to the template molecule may be polymerized without limitation.

Specifically, the first adapter nucleic acid molecule may be in the form of DNA, but is not limited thereto.

Additionally, specifically, the first adapter nucleic acid molecule may have its 5' end adenylated, but is not limited thereto.

Also specifically, the first adapter nucleic acid molecule may be one which has a modified 3' end to prevent the ligation of another adapter molecule to its 3' end, and may be, for example, amine modified, but is not limited thereto.

As used herein, "a second adapter nucleic acid molecule" is a nucleic acid molecule that is ligated to the 3' end of the cDNA synthesized through complementary strand polymerization reaction using a molecule, in which a first adapter nucleic acid molecule is ligated to the 3' end of target RNA, as a template. A phosphate group at the 5' end of the second adapter nucleic acid molecule recognizes and binds to the 3' end of the cDNA.

Specifically, the second adapter nucleic acid molecule may be in the form of DNA, but is not limited thereto.

Also specifically, the second adapter nucleic acid molecule may be one which has an adenylated 5' end, but is not limited thereto.

Also specifically, the second adapter nucleic acid molecule may be one which has a modified 3' end to prevent the ligation of another adapter molecule to its 3' end, and may be, for example, amine modified, but is not limited thereto.

Specifically, the complementary strand polymerization reaction of the step b) may be performed using DNA polymerase, but is not limited thereto, and any reverse transcriptase or polymerase used in a conventional reverse transcription reaction may be applied.

Also specifically, the terminus of the cDNA may include an adenine (A) base through the complementary strand polymerization reaction.

Also specifically, there may be added a step of treating the guide strand simultaneously with the step d), or after the step d) and before the PCR amplification reaction, to thereby increase the ligation efficiency of a second adapter nucleic acid molecule to the 3' end of the cDNA.

As used herein, "guide strand" is a strand in which one part complementarily binds to the cDNA and the other part complementarily binds to a second adapter nucleic acid molecule.

Accordingly, the guide nucleic acid molecule strand includes a sequence complementary to the 3' end of the cDNA and the 5' end of a second adapter nucleic acid molecule. More specifically, the guide strand may include a thymine (T) base, and the thymine may complementarily bind to adenine at the end of the cDNA.

More specifically, the guide strand may complementarily bind to the 3' end of the cDNA and the 5' end of the second adapter nucleic acid molecule. Through this binding, the guide strand plays a supporting role in the ligation to the 3' end of cDNA and the 5' end of the second adapter nucleic acid molecule. As the sequence of the guide strand of the present invention, any sequence capable of complementarily binding to the 3' end of the cDNA and the 5' end of the second adapter nucleic acid molecule may be applied without limitation.

The guide strand may include about 10 to about 20 bases, and more specifically, may include about 10 bases, but is not limited thereto. The length of the guide strand of the present invention may be applied after adjustment as needed, as long as the guide strand can stably ligate the 3' end of the cDNA generated in the process of constructing the RNA library of the present invention and the 5' end of the second adapter nucleic acid molecule.

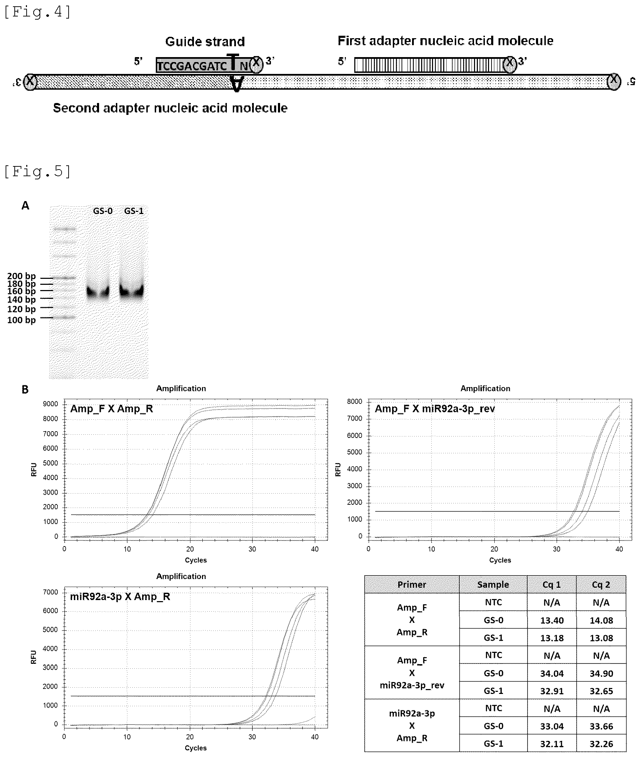

In an embodiment of the present invention, it was confirmed that when the guide strand is bound, the efficiency of ligating the second adapter nucleic acid molecule to the 3' end of the cDNA is improved (FIG. 5). Through this, RNA library construction efficiency can be further improved.

Another aspect of the present invention relates to a composition for constructing an RNA sample library, which composition includes: a) a first adapter nucleic acid molecule to be ligated to the 3' end of a target RNA; b) a second adapter nucleic acid molecule to be bound to the 3' end of the cDNA synthesized through the complementary strand polymerization of the ligated molecule; and c) the guide strand.

The first adapter nucleic acid molecule, the second adapter nucleic acid molecule and the guide strand are as described above.

The composition for constructing an RNA library may further include a buffer, PCR primers, etc., and the components to be further included may be applied after adjustment as needed.

Still another aspect of the invention relates to an RNA library constructed by the construction method described above. RNAs related to individual diseases, changes in conditions, etc. may be pre-designed in the RNA library constructed as described above, and this library can be utilized for screening and development of genetic markers and therapeutic candidate substances for disease treatment or improvement of conditions.

### ADVANTAGEOUS EFFECTS

The RNA library construction method of the present invention includes the steps of ligating the first adapter nucleic acid molecule and the second adapter nucleic acid molecule, and thus can improve the construction efficiency and shorten the construction time while maintaining accuracy compared to the conventional RNA library construction methods. Furthermore, the efficiency of RNA library construction can be improved by increasing the ligation efficiency of adapter nucleic acid molecules through the addition of guide nucleic acid molecules.

The effects of the present invention are not limited to the above-described effects, and it should be understood that all of the effects that can be derived from the constitutions described in the detailed description of the present invention or the inventions described in the claims.

### BRIEF DESCRIPTION OF THE DRAWINGS

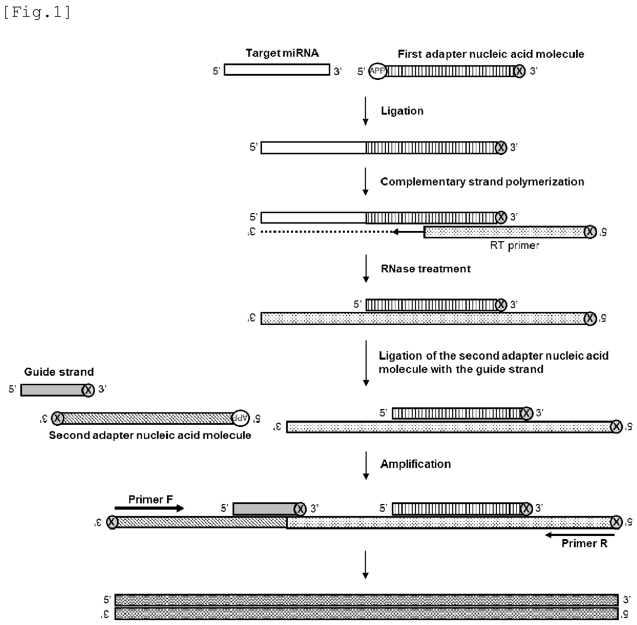

FIG. 1 is a schematic diagram illustrating the RNA library construction of the present invention. The X mark at the 3' end in the schematic diagram indicates that an amine region is modified to block additional binding.
FIG. 2 shows the results of confirming the library constructed by the method of the present invention through electrophoresis.
FIG. 3 shows the results of confirming the library constructed by the method of the present invention through qPCR (NTC: negative control, Library: the library constructed by the present invention).
FIG. 4 is a schematic diagram illustrating the binding of the guide strand of the present invention in the ligation of a second adapter nucleic acid molecule to the 3' end of cDNA. The X mark at the 3' end in the schematic diagram indicates that an amine region is modified to block additional binding.
FIG. 5 shows the results of confirming the library construction efficiency according to the guide strand binding (FIG. 5A: electrophoresis results of GS-0 and GS-1, FIG.5B: qPCR comparison results between GS-0 and GS-1).

### MODE FOR CARRYING OUT THE INVENTION

Hereinafter, the present invention will be described in detail by examples. However, the following examples are only intended to illustrate the present invention, and the present invention is not limited to the following examples.

### Example1. Construction of RNA library

### 1-1. RNA extraction

Small RNA was extracted from the blood using the XENOPURE blood small RNA purification kit (Xenohelix) according to the product instructions.

### 1-2. Ligation of small RNA and first adaptor nucleic acid molecule

The small RNA extracted from the blood was ligated to a first adapter nucleic acid molecule with an adenylated 5' end using T4 RNA ligase 2, truncated KQ kit (NEB). The extracted small RNA (3 µL), first adapter nucleic acid molecule with an adenylated 5' end (1 µL; 1 pmol/µL), 10X T4 RNA ligase reaction buffer (NEB) (2 µL), 50% PEG8000 (NEB) (10 µL), T4 RNA ligase 2, truncated KQ(NEB) (1 µL), RNase inhibitor (1 µL, Takara), and RNase/DNase free water (2 µL) were mixed, and then reacted using a PCR device at 28°C for 60 minutes, at 65°C for 20 minutes, and at 4°C for 5 minutes.

### 1-3. Complementary strand polymerization

cDNA of small RNA, to which a first adapter nucleic acid molecule is ligated, was synthesized using a DNA polymerase kit (Xenohelix). To a small RNA (20 µL), to which a first adapter nucleic acid molecule is ligated, 10X PCR buffer (4 µL, Xenohelix), 10 mM dNTP (4 µL, Xenohelix), DNA polymerase (1 µL, Xenohelix), RNase inhibitor (1 µL, Takara), primers with an index sequence (1 µL; 1 pmol/µL), and RNase/DNase free water(9 µL) were added and mixed, and then reacted using a PCR device at 95°C for 30 seconds, and at 60°C for 10 minutes.

### 1-4. RNase treatment

RNA was degraded using the Thermostable RNase H kit (Enzynomics). To the RNA/DNA chimera product (40 µL) synthesized by the complementary strand polymerization reaction, 10X RNase H buffer (10 µL, Enzynomics), Thermostable RNase H (1 µL, Enzynomics), and RNase/DNase free water (49 µL) were added and mixed, and then reacted at 50°C for 20 minutes using a PCR device. Upon completion of the reaction, a cleanup procedure was performed using a PCR purification kit (Xenohelix) according to the product manual.

### 1-5. Ligation of second adapter nucleic acid molecule

A second adapter nucleic acid molecule with an adenylated 5' end was ligated using the Thermostable 5'App DNA/RNA Ligase kit (NEB). To the product (37 µL), which was cleaned after treating with RNase H, 10x NE Buffer 1 (NEB) (5 µL), 50 mM MnCl₂ (5 µL, NEB), thermostable 5' App DNA/RNA ligase (2µL, NEB), a second adapter nucleic acid molecule with an adenylated 5' end (1 µL, 1 pmol/µL),and a guide strand (1 µL, 1 pmol/uL) were added and mixed, and then reacted using a PCR device at 65°C for 60 minutes, and at 90°C for 3 minutes. Upon completion of the reaction, a cleanup procedure was performed using a PCR purification kit (Xenohelix) according to the product manual.

### 1-6. Amplification

Amplification was performed using the HotStart Taq DNA Polymerase kit (Xenohelix). To the product (37 µL), which was cleaned after ligating to a second adapter nucleic acid molecule, 10X reaction buffer (8 µL, Xenohelix), 10 nM dNTP(8 µL, Xenohelix), HotStart Taq DNA Polymerase(1 µL, Xenohelix), forward primer (1 µL, 10 pmol/µL), reverse primer (1 µL, 10 pmol/µL), and RNase/DNase free water (24 µL) were added and mixed, and then reacted using a PCR device under the following conditions: 1 cycle of 95°C for 15 minutes; 22 cycles of 95°C for 15 seconds, 60°C for 30 seconds, 72°C for 15 seconds, 1 cycle of 72°C for 2 minutes, and 1 cycle of 4°C for 5 minutes. Upon completion of the reaction, a cleanup procedure was performed using a PCR purification kit (Xenohelix) according to the product manual.

### 1-7. Confirmation of completed library

The completion of the RNA library constructed through the above process was confirmed through electrophoresis. Specifically, the RNA library prepared was electrophoresed on a 6% polyacrylamide gel (19:1) and the completed library was confirmed using the Gel Doc.

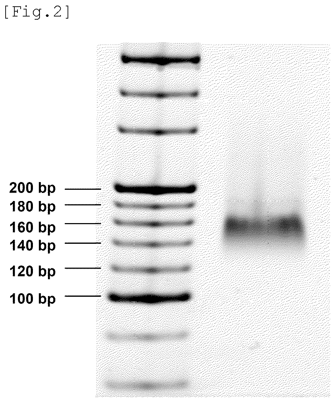

As a result, as shown in FIG. 2, the length of the completed library was about 140 bp to about 150 bp, and a band near a size of about 150 bp was observed in the electrophoresis gel, thus confirming that the miRNA library was successfully constructed. Furthermore, the dimer band (120-130 bp) of the first adapter and the second adapter did not appear, thus confirming that there was no non-specific reaction due to the adapter nucleic acid molecule used in the present invention.

Additionally, qPCR was performed using primers made from the sequences of both ends of the library. Specifically, 9 µL of TB Green Premix Ex Taq II (Takara), 2 µL of Primer mix (0.5 pmol/µL each), and 12 µL of RNase/DNase free were added to the library (2 µL), which was diluted to a concentration of 1/500, and mixed, and reacted using a real-time PCR device under the following conditions: 1 cycle of 95°C for 3 minutes; and 40 cycles of 95°C for 10 seconds, and 64°C for 30 seconds.

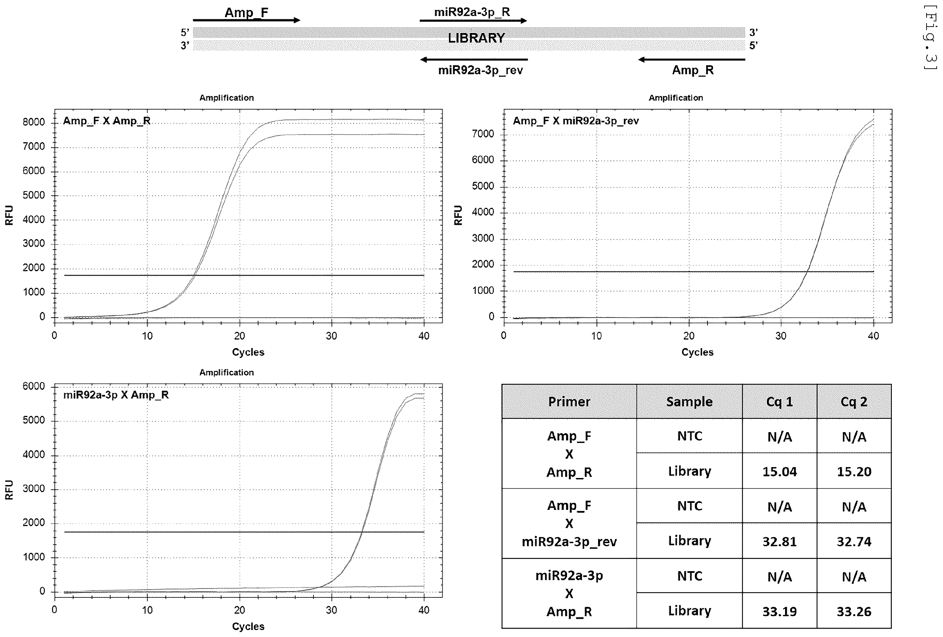

As a result, as shown in FIG. 3, it was confirmed that the library was successfully constructed with a Cq value of about 15. In addition, as a result of qPCR using the primers made from both ends of the library and the miR92a-3p sequence, the Cq values were about 32 and about 33, thus confirming that the first and second adapter nucleic acid molecules were successfully ligated to the miRNA to thereby complete the library.

### Example 2. Confirmation of library construction efficiency according to guide strand binding

In the ligation of the second adapter nucleic acid molecule to the 3' end of the cDNA synthesized during the library construction process, the efficiency of adding a guide strand was confirmed.

The guide strand was prepared to include i) a base sequence complementary to the 5' end of the second adapter nucleic acid molecule, ii) thymine (T), a base complementary to the A tail of the cDNA generated using polymerase, and iii) a base N that can bind to all of A, C, G, and T. The sequence of the exemplary guide strand in the present invention is identical to SEQ ID NO: 4 listed in Table 3 below. In addition, a schematic diagram of how a guide strand binds to the 3' end of cDNA in the ligation of a second adapter nucleic acid molecule is as shown in FIG. 4.

An RNA library was constructed according to the construction method of Example 1, in which the library was constructed such that it does not include the guide strand added in Example 1-5 above (GS-0), and that it includes the guide strand in Example 1-5 above (GS-1), thereby confirming the libraries of GS-0 and GS-1, respectively.

As a result, as shown in FIG. 5A, bands around the size of about 150 bp were observed for both GS-0 and GS-1, and the dimer bands (120-130 bp) of the first and second adapters did not appear, thus confirming that an miRNA library was successfully constructed.

Additionally, qPCR was performed in the same manner as in Example 1-7 above to confirm the completed library. qPCR was performed using primers made using both ends of the library and the miR92a-3p sequence.

As a result, as shown in FIG. 5B, it was confirmed that the Cq value of GS-1 containing the guide strand was decreased compared to GS-0 containing no guide strand.

As described above, in the library construction method of the present invention, it was confirmed that the addition of a guide strand can increase the ligation efficiency of the second adapter nucleic acid molecule to the 3' end of cDNA and increase the construction efficiency of an RNA library.

### Example 3. Confirmation of library constructed using synthetic miRNA 92a-3p

As an example, a library was constructed according to the library construction method of the present invention using synthetic miRNA 92a-3p, and then the constructed library was confirmed by sequencing.

As a result of the analysis of the reads after trimming the read length to 16-30 bp according to the miRNA, which has a length of 21-23 nt, 3,122,512 among the total 3,534,990 reads were mapped to human miRNA data, as shown in Table 1 below.

**[Table 1]**

| Sample | Total reads | Number of reads mapped to miRNA | Number of detected miRNA |
|---|---|---|---|
| Library | 3,534,990 | 3,122,512 | 2 |

Furthermore, as shown in Table 2 below, the detected miRNAs were has-miR-92a-3p and has-miR-92b-3p, and most of the reads were has-miR-92a-3p, thus confirming that the library was successfully constructed.

**[Table 2]**

| No. | miRNA | Read counts |
|---|---|---|
| 1 | hsa-miR-92a-3p | 3, 122, 038 |
| 2 | hsa-miR-92b-3p | 474 |

Each adapter nucleic acid molecule, guide strand, and primer sequences used in Examples 1 to 3 above are summarized in Table 3 below. The primer sequences of the present invention can be synthesized using the nucleic acid sequence of an adapter nucleic acid molecule regardless of the type of target nucleic acid molecule to be amplified.

**[Table 3]**

| SEQ ID NO: | Category | Sequence (5'-3') | Use |
|---|---|---|---|
| 1 | First adaptor nucleic acid molecule | TGGAATTCTCGGGTGCCAAGGAACTCCAG TCAC | ligation to target RNA |
| 2 | Second adaptor nucleic acid molecule | GATCGTCGGACTGTAGAACTCTGAACGTG TAGATCTCGGTGGTCGCCGTATCATT | ligation to cDNA |
| 3 | Complementar y strand polymerizati on primer | CAAGCAGAAGACGGCATACGAGATCTGAT CGTGACTGGAGTTCCTTGGCACC | complementary strand polymerization |
| 4 | Guide strand | TCCGACGATCTN | conjugation of cDNA and second adapter nucleic acid molecule |
| 5 | Amp forward primer (Amp_F) | AATGATACGGCGACCACCGAGA | amplification and confirmation of library |
| 6 | Amp reverse primer (Amp_R) | CAAGCAGAAGACGGCATACGAGAT | amplification and confirmation of library |
| 7 | miR92a-3p forward primer (miR92a-3p) | TATTGCACTTGTCCCGGCCT | confirmation of library |
| 8 | miR92a-3p reverse primer (miR92a-3p_Rev) | ACAGGCCGGGACAAGTGCAATA | confirmation of library |

The above description of the present invention is for illustrative purposes, and those of ordinary skill in the art to which the present invention pertains will understand that it can easily be modified into other specific forms without changing the technical idea or essential features of the present invention. Therefore, it should be understood that the examples described above are illustrative in all respects and not restrictive. For example, each component described as a single entity may also be implemented in a distributed manner, and likewise, components described as distributed may be implemented in a combined manner.

The scope of the present invention is indicated by the claims set forth below, and all changes or modifications derived from the meaning and scope of the claims and their equivalent concepts should be interpreted as being included in the scope of the present invention.

## Claims

1. A method for constructing an RNA library, the method comprising:
a) ligating a first adapter nucleic acid molecule to a 3' end of a target RNA;
b) synthesizing cDNA through complementary strand polymerization of the molecule ligated in the step a);
c) removing RNA by treating with RNase;
d) ligating a second adapter nucleic acid molecule to the 3' end of the cDNA; and
e) PCR amplifying the nucleic acid molecule ligated in the step d).

2. The method of claim 1, wherein the target RNA is a small RNA.

3. The method of claim 2, wherein the small RNA is an miRNA.

4. The method of claim 1, wherein the first adapter nucleic acid molecule is in the form of DNA.

5. The method of claim 1, wherein the first adapter nucleic acid molecule is adenylated at the 5' end.

6. The method of claim 1, wherein the second adapter nucleic acid molecule is in the form of DNA.

7. The method of claim 1, wherein the second adapter nucleic acid molecule is adenylated at the 5' end.

8. The method of claim 1, wherein the complementary strand polymerization in the step b) uses a DNA polymerase.

9. The method of claim 1, wherein the 3' end of the cDNA comprises an adenine (A) base.

10. The method of claim 1, further comprising treating a guide strand, either simultaneously with the step d) or after the step d) but before the PCR amplification reaction, to increase the ligation efficiency of the second adapter nucleic acid molecule to the 3' end of the cDNA.

11. The method of claim 10, wherein the guide nucleic acid molecule strand comprises sequences complementary to the 3' end of the cDNA and the 5' end of the second adapter nucleic acid molecule.

12. The method of claim 10, wherein the guide nucleic acid molecule strand comprises a thymine (T) base.

13. The method of claim 10, wherein the guide nucleic acid molecule strand complementarily binds to the 3' end of the cDNA and the 5' end of the second adapter nucleic acid molecule.

14. A composition for constructing an RNA library, the composition comprising:
a) a first adapter nucleic acid molecule to be ligated to a 3' end of target RNA;
b) a second adapter nucleic acid molecule to be ligated to a 3' end of the cDNA that has been synthesized through the complementary strand polymerization of the ligated molecule; and
c) the guide strand.

15. An RNA library constructed by the method of claim 1.
